(19) Europäisches Patentamt
European Patent Office
Office européen des brevets

(11) Publication number: 0 083 857 B1

(12) EUROPEAN PATENT SPECIFICATION

(45) Date of publication of patent specification: 05.06.85

(51) Int. Cl.⁴: C 12 Q 1/36, C 07 K 5/06

(21) Application number: 82306707.9

(22) Date of filing: 15.12.82

# (54) Derivatives of hippuryl-L-phenylalanine and their use in determining the activity of carboxypeptidase A.

(30) Priority: 16.12.81 JP 201587/81

(43) Date of publication of application: 20.07.83 Bulletin 83/29

(45) Publication of the grant of the patent: 05.06.85 Bulletin 85/23

(84) Designated Contracting States: DE FR GB NL

(56) References cited:
CLINICAL CHEMISTRY, Vol. 27, No. 11, November 1981, Y.KASAHARA et al. "Colorimetry of angiotensin-1 converting enzyme activity in serum", pages 1922- 1925

Chemical Abstracts vol. 68, no. 19, 6 May 1968, Columbus, Ohio, USA, R.C. DAVIES et al. "Kinetics of carboxypeptidase A. I. Hydrolysis of carbobenzoxyglycyl-L-phenylalanine, benzoylglycyl-L-phenylalanine, and hippuryl-DL-beta-phenyllactic acid by metal-substituted and acetylated carboxypeptidases", page 8134, column 1, abstract no. 84577h

(73) Proprietor: FUJIREBIO KABUSHIKI KAISHA also trading as FUJIREBIO INC.
6-7, Shimoochiai 4-chome Shinjuku-ku
Tokyo 161 (JP)

(72) Inventor: Sugiyama, Masami
9-19 Motoyokoyamamachi 2-chome
Hachioji-shi Tokyo (JP)
Inventor: Kasahara, Yasushi
310, 4-4, Nagayama 3-chome
Fuchu-shi Tokyo (JP)
Inventor: Ashihara, Yoshihiro
28 Harumicho 1-chome
Fuchi-shi Tokyo (JP)

(74) Representative: Silverman, Warren et al
HASELTINE LAKE & CO. Hazlitt House 28
Southampton Buildings Chancery Lane
London WC2A 1AT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 083 857 B1

**Description**

This invention relates to derivatives of hippuryl-L-phenylalanine and to their use as substrates for measuring the activity of carboxypeptidase A (hereinafter referred to as CP—A for short).

CP—A is a protein-decomposing enzyme which is found in the pancreas and the blood serum. The activity of CP—A depends on the disease which is present and on its extent. Accordingly, by measuring the activity of the CP—A, the extent to which a disease has spread can be measured.

According to one aspect of the invention, there is provided a derivative of hippuryl-L-phenylalanine having the following general formula

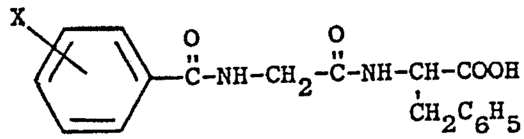

(I)

wherein

X represents OH or $CH_3O$.

According to a second aspect of the invention, there is provided a method for determining the activity of carboxypeptidase A comprising the steps of

(1) bringing into contact with each other in a liquid medium containing carboxypeptidase A as substrate, (i) a derivative of hippuryl-L-phenylalanine having the following general formula:

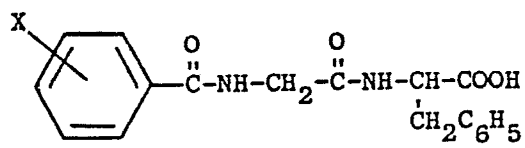

wherein

X represents OH or $CH_3O$

(ii) hippuricase, (iii) 4-aminoantipyrine and (iv) an oxidising agent capable as such of oxidising X-benzoic acid produced *in situ* to yield a substance which reacts with 4-aminoantipyrine present to yield a quinonimine dye;

(2) colourimetrically measuring the concentration of the quinonimine dye and

(3) calculating the activity of said enzyme from the concentration of the quinonimine dye.

More particularly, the activity of CP—A can be measured by using the derivatives of this invention, which are hereinafter termed X-hippuryl-L-phenylalanines as follows:

X-hippuryl-L-phenylalanine is added to a liquid containing CP—A, such as human blood serum or humour, to produce X-hippuric acid and L-phenylalanine as a result of its decomposition by the CP—A. Hippuricase present in or added to the liquid produces X-benzoic acid and glycine by decomposing the X-hippuric acid which has been produced. Quinonimine dye is produced by the reaction of X-benzoic acid with 4-aminoantipyrine in the presence of an oxidizing agent. The concentration of the quinonimine dye is colourimetrically measured to indicate the activity of CP—A. If desired the substrate may be first reacted with a mixture of the hippuryl-L-phenylalanine hippuricase and 4-amino-antipyrine and the reaction product obtained is mixed with the oxidising agent.

Finally, in a third aspect, this invention provides a diagnostic composition for determining the activity of carboxypeptidase A which comprises (i) a derivative of hippuryl-L-phenylalanine having the following general formula:

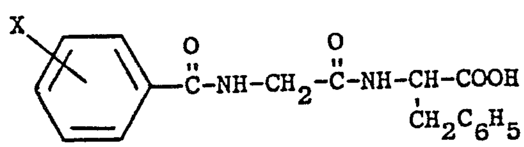

wherein

X represents OH or $CH_3O$, (ii) hippuricase, (iii) 4-aminoantipyrine and (iv) an oxidising agent capable as such of oxidising X-benzoic acid to yield a substance which reacts with 4-aminoantipyrine to yield a quinonimine dye.

When the derivatives of hippuryl-L-phenylalanine according to this invention are used in determining the activity of CP—A, the oxidising agent used is for example, periodic acid or a periodate, perchloric acid

or a perchlorate, potassium ferricyanide, ammonium ceric nitrate, chloramine T, chloramine B or chromium trioxide.

Once the concentration of the quinonimine dye is known, the activity unit (mU) of CP—A can be calculated by the following equation:

$$mU = \frac{A-B}{\text{Molecular extinction coefficient } (\varepsilon)} \times \frac{1}{\text{Reaction time (20 min.)}} \times \frac{1}{\text{Light path length (cm)}} \times \frac{1.05}{0.05} \times 10^6$$

where $\varepsilon = 12\ 000$ (Molecular extinction coefficient of quinonimine dye)

A is the absorbance of the reaction mixture and

B is the absorbance of a blank test reagent, both absorbances being measured at the same wavelength, preferably 505 nm.

The derivatives of hippuryl-L-phenylalanine can be prepared by the following reaction:

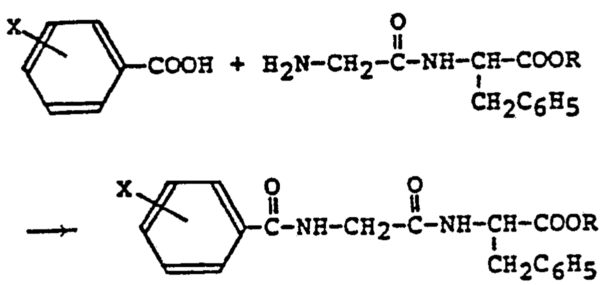

(II)

wherein

X represents OH or $CH_3O$, and R represents an alkyl group.

This reaction is generally carried out in an organic solvent such as dicyclohexylcarbodiimide, and the compound of general formula (I) can be obtained by hydrolysis of the compound having general formula (II).

The following preparative Examples are given by way of illustration only:

Example 1

Synthesis of p-hydroxyhippuryl-L-phenylalanine:

20.9 g (0.1 M) of carbobenzoxyglycine were dissolved in 500 ml of dichloromethane and 19.3 g (0.1 M; of L-phenylalanine ethyl were added to this solution after which the mixture obtained was cooled in ice-water to 0°C and 20.6 g (0.1 M) of dicyclohexylcarbodiimide were added. The mixture was then stirred overnight.

The deposit which had formed was filtered off using a glass filter, and after the filtrate had been washed with a 0.1% by weight aqueous solution of $NaHCO_3$ three times and then with a 0.1 N by weight aqueous solution of HCl three times, it was dried over anhydrous magnesium sulphate and freed of dichloromethane under reduced pressure to obtain carbobenzoxyglycyl-L-phenylalanine ethyl ester. 250 ml of HBr-saturated acetic acid were added, and after the mixture had been stirred for 30 minutes, anhydrous ether was added to the mixture to produce as precipitate. The precipitate was filtered off and then washed with ether to obtain glycyl-L-phenylalanine ethyl ester HBr.

16.5 g (0.05 M) of glycyl-L-phenylalanine ethyl ester .HBr, 9.0 g (0.05 M) of p-acetoxybenzoic acid and 7.5 ml (0.05 M) of triethylamine were dissolved in 250 ml of dichloromethane, and after this solution had been cooled to 0°C and 10.3 g (0.05 M) of dicyclohexylcarbodiimide had been added the reaction mixture obtained was stirred for 24 hours. The precipitate produced was removed, and after the filtrate had been washed with a 0.1% aqueous solution of $NaHCO_3$ and then with a 0.1 N aqueous solution of HCl, it was dried over anhydrous magnesium sulphate. Dichloromethane was then distilled off under reduced pressure to leave p-acetoxyhippuryl-L-phenylalanine ethyl ester behind.

7.96 g (0.02 M) of p-acetoxyhippuryl-L-phenylalanine ethyl ester were dissolved in 50 ml of methanol, and to this solution were added 70 ml of a 0.1 N aqueous solution of NaOH. After the solution obtained had been stirred at ambient temperature for one hour, the pH of the solution was adjusted to 7 by adding HCl. The solution was then subjected to distillation under reduced pressure to remove the solvent and methanol was added to the residue. Insoluble matter was filtered off, and ethanol was then added to the filtrate to produce a precipitate. The filtered off precipitate consisted of p-hydroxyhippuryl-L-phenylalanine, which was obtained in an amount of 4.01 g. m.p. 163—167.5°C (decomposed).

Referring to the accompanying drawings, Figure 1 shows the H-nmr absorption spectrum of p-hydroxyhippuryl-L-phenylalanine, and Figure 2 shows its infrared absorption spectrum.

Example 2

Synthesis of o-hydroxyhippuryl-L-phenylalanine:

Glycyl-L-phenylalanine ethyl ester .HBr was obtained in the manner indicated in Example 1 using carbobenzoxyglycine as starting material.

16.5 g (0.05 M) of glycyl-L-phenylalanine ethyl ester .HBr, 9.0 g (0.05 M) of o-acetoxybenzoic acid and 7.5 ml (0.05 M) of triethylamine were dissolved in 250 ml of dichloromethane, and after this solution had been cooled to 0°C, 10.3 g (0.05 M) of dicyclohexylcarbodiimide were added and the mixture obtained was stirred for 24 hours. The precipitate which formed was removed, and after the filtrate had been washed with a 0.1% by weight aqueous solution of $NaHCO_3$ and then with a 0.1 N aqueous solution of HCl, and was dried over anhydrous magnesium sulphate, dichloromethane was distilled off under reduced pressure to obtain o-acetoxyhippuryl-L-phenylalanine ethyl ester.

7.96 g (0.02 M) of o-acetoxyhippuryl-L-phenylalanine ethyl ester were dissolved in 50 ml of methanol and to this solution were added 70 ml of a 0.1 N aqueous solution of NaOH, and then this solution was worked up in the same manner as the corresponding solution in Example 1 to yield o-hydroxyhippuryl-L-phenylalanine.

Example 3

Synthesis of p-methoxyhippuryl-L-phenylalanine:

p-Methoxyhippuryl-L-phenylalanine was obtained by repeating the procedure of Example 1, but using 7.4 g (0.05 M) of p-methoxybenzoic acid instead of 9.0 g (0.05 M) of p-acetoxybenzoic acid.

**Claims**

1. Derivatives of hippuryl-L-phenylalanine having the following general formula:

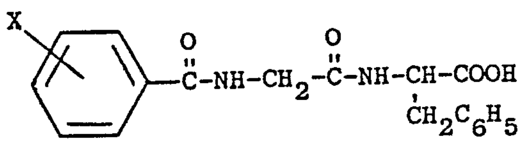

(I)

wherein

X represents OH or $CH_3O$.

2. p-Hydroxyhippuryl-L-phenylalanine.

3. o-Hydroxyhippuryl-L-phenylalanine.

4. p-Methoxyhippuryl-L-phenylalanine.

5. A method for determining the activity of carboxypeptidase A comprising the steps of

(1) bringing into contact with each other in a liquid medium containing carboxypeptidase A as substrate, (i) a derivative of hippuryl-L-phenylalanine having the following general formula:

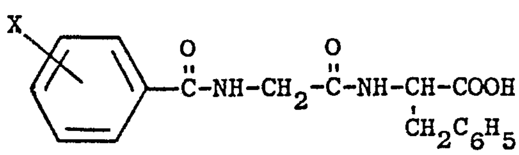

wherein

X represents OH or $CH_3O$ (ii) hippuricase, (iii) 4-aminoantipyrine and (iv) an oxidising agent capable as such of oxidising X-benzoic acid produced *in situ* to yield a substance which reacts with 4-aminoantipyrine present to yield a quinonimine dye;

(2) colourimetrically measuring the concentration of the quinonimine dye and

(3) calculating the activity of said enzyme from the concentration of the quinonimine dye.

6. A method as claimed in claim 5, wherein the oxidising agent is periodic acid or a periodate, perchloric acid or a perchlorate, potassium ferricyanide ammonium ceric nitrate, chloramine-T, chloramine-B or chromium trioxide.

7. A method as claimed in claim 5 or 6, which is applied to the determination of the activity of carboxypeptidase A in human blood serum or humour.

8. A diagnostic composition for determining the activity of carboxypeptidase A which comprises (i) a derivative of hippuryl-L-phenylalanine having the following general formula:

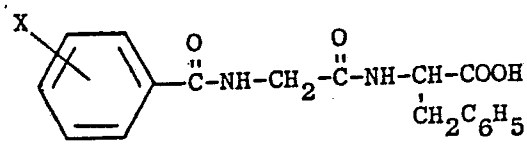

wherein

X represents OH or $CH_3O$, (ii) hippuricase, (iii) 4-aminoantipyrine and (iv) an oxidising agent capable as such of oxidising X-benzoic acid to yield a substance which reacts with 4-aminoantipyrine to yield a quinonimine dye.

9. A composition as claimed in claim 8, wherein the oxidising agent is periodic acid or a periodate, perchloric acid or a perchlorate, potassium ferricyanide ammonium ceric nitrate, chloramine-T, chloramine-B or chromium trioxide.

**Patentansprüche**

1. Derivate des Hippuryl-L-phenylalanins mit der folgenden allgemeinen Formel

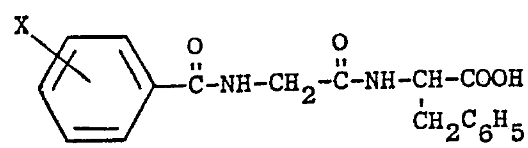

worin X OH oder $CH_3O$ bedeutet.

2. p-Hydroxyhippuryl-L-phenylalanin.

3. o-Hydroxyhippuryl-L-phenylalanin.

4. p-Methoxyhippuryl-L-phenylalanin.

5. Verfahren zur Bestimmung der Aktivität von Carboxypeptidase A, umfassend die folgenden Stufen:

(1) das in Berührung bringen in einem flüssigen, Carboxypeptidase A als Substrat enthaltenden Medium von (i) einem Derivat des Hippuryl-L-phenylalanins mit der folgenden algemeinen Formel

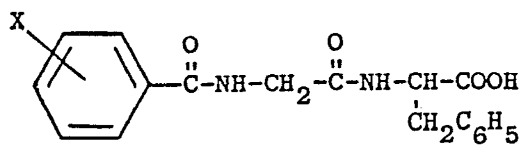

worin X =H oder $CH_3O$ bedeutet, (ii) Hippuricase, (iii) 4-Aminoantipyrin und (iv) einem oxidierenden Mittel, das fähig ist, die in situ gebildete, durch den X-Rest substituierte Benzoesäure zu einer Substanz zu oxidieren, die mit dem vorhandenen 4-Aminoantipyrin unter Bildung eines Chinoniminfarbstoffs reagiert,

(2) kolorimetrische Messung der Konzentration des Chinoniminfarbstoffs und

(3) Berechnung der Aktivität des genannten Enzyms aus der Konzentration des Chinoniminfarbstoffs.

6. Verfahren nach Patentanspruch 5, worin das oxidierende Mittel Perjodsäure oder ein Perjodat, Perchlorsäure oder ein Perchlorat, Kaliumferricyanid, Ammoniumcernitrat, Chloramin-T, Chloramin-B oder Chromtrioxid ist.

7. Verfahren nach Patentanspruch 5 oder 6, welches angewandt wird zur Bestimmung der Aktivität von Carboxypeptidase A in menschlichem Blutserum oder Flüssigkeit.

8. Eine diagnostische Mischung zur Bestimmung der Aktivität von Carboxypeptidase A, bestehend aus (i) einem Derivat vom Hippuryl-L-phenylalanin mit der folgenden allgemeinen Formel

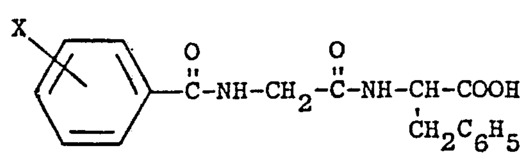

worin X OH oder $CH_3O$ bedeutet, (ii) Hippuricase, (iii) 4-Aminoantipyrin und (iv) einem oxidierenden Mittel,

das fähig ist, die durch den X-Rest substituierte Benzoesäure zu einer Substanz zu oxidieren, die mit dem 4-Aminoantipyrin unter Bildung eines Chinoniminofarbstoffs reagiert.

9. Eine Mischung nach Patentanspruch 8, worin das oxidierende Mittel Perjodsäure oder ein Perjodat, Perchlorsäure oder ein Perchlorat, Kaliumferricyanid, Ammoniumcernitrat, Chloramin-T, Chloramin-B oder Chromtrioxid ist.

**Revendications**

1. Dérivés d'hippuryl-L-phénylalanine répondant à la formule générale

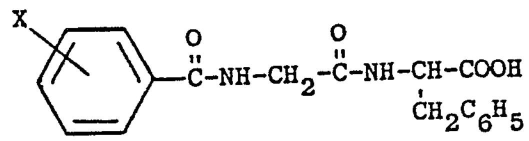

dans laquelle X représente OH ou $CH_3O$.

2. p-hydroxyhippuryl-L-phénylalanine.

3. o-hydroxyhippuryl-L-phénylalanine.

4. p-méthoxy-hippuryl-L-phénylalanine.

5. Procédé de détermination de l'activité de la carboxypeptidase-A qui consiste:

(1) à mettre en contact mutuel dans un milieu liquide contenant la carboxypeptidase-A, à titre de substrat, (i) un dérivé de l'hippuryl-L-phénylalanine répondant à la formule générale:

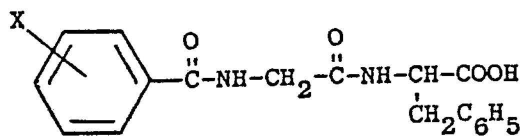

dans laquelle X représente OH ou $CH_3O$, (ii) l'hippuricase, (iii) la 4-aminoantipyrine et (iv) un agent oxydant capable d'oxyder l'acide X-benzoïque produit *in situ* pour donner une substance qui réagit avec la 4-aminoantipyrine présente pour obtenir un colorant de quinone-imine;

(2) à mesurer par voie colorimétrique la concentration du colorant de quinone-imino; et

(3) à calculer l'activité de ladite enzyme à partir de la concentration du colorant de quinone-imine.

6. Procédé selon la revendication 5, dans lequel l'agent oxydant est l'acide periodique ou un periodate, l'acide perchlorique ou un perchlorate, le ferricyanure de potassium, le cérinitrate d'ammonium, la chloramine-T, la chloramine-B ou le trioxyde de chrome.

7. Procédé selon la revendication 5 ou 6, que est appliqué à la détermination de l'activité de la carboxypeptidase-A dans le sérum sanguin humain ou humeur.

8. Composition de diagnostic pour déterminer l'activité de la carboxypeptidase-A, qui comprend (i) un dérivé de l'hippuryl-L-phénylalanine répondant à la formule générale suivante:

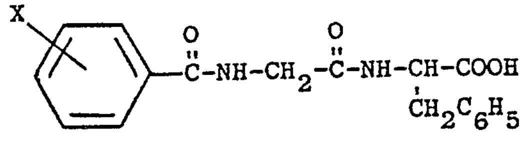

dans laquelle X représente OH ou $CH_3$, (ii) l'hippuricase, (iii) la 4-aminoantipyrine et (iv) un agent oxydant capable d'oxyder l'acide X-benzoïque pour obtenir une substance qui réagit avec la 4-aminoantipyrine pour produire un colorant de quinone-imine.

9. Composition selon la revendication 8, dans laquelle l'agent oxydant est l'acide periodique ou un periodate, l'acide perchlorique ou un perchlorate, le ferricyanure de potassium, le cérinitrate d'ammonium, la chloramine-T, la chloramine-B ou le trioxyde de chrome.

FIG. 1

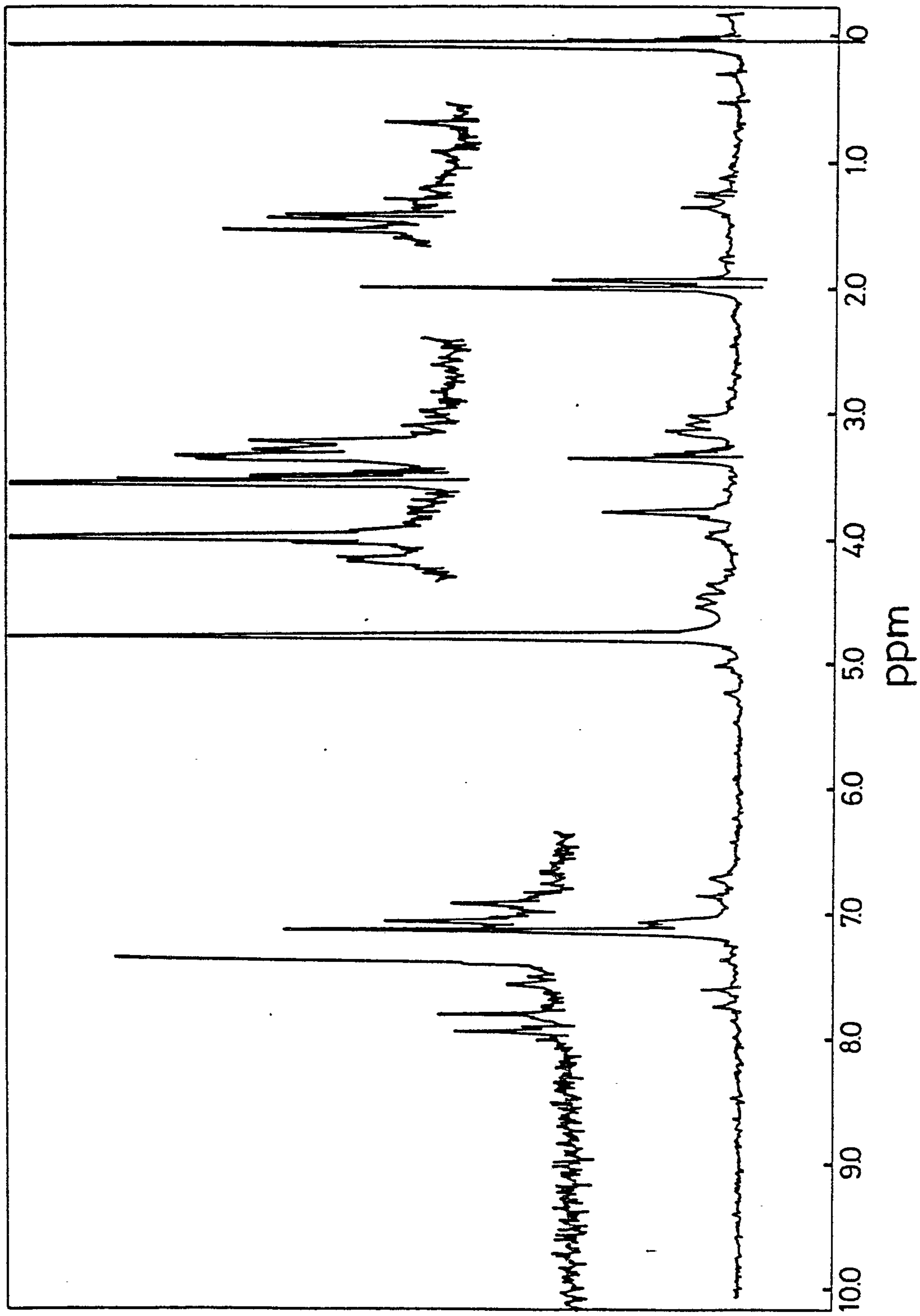

FIG. 2

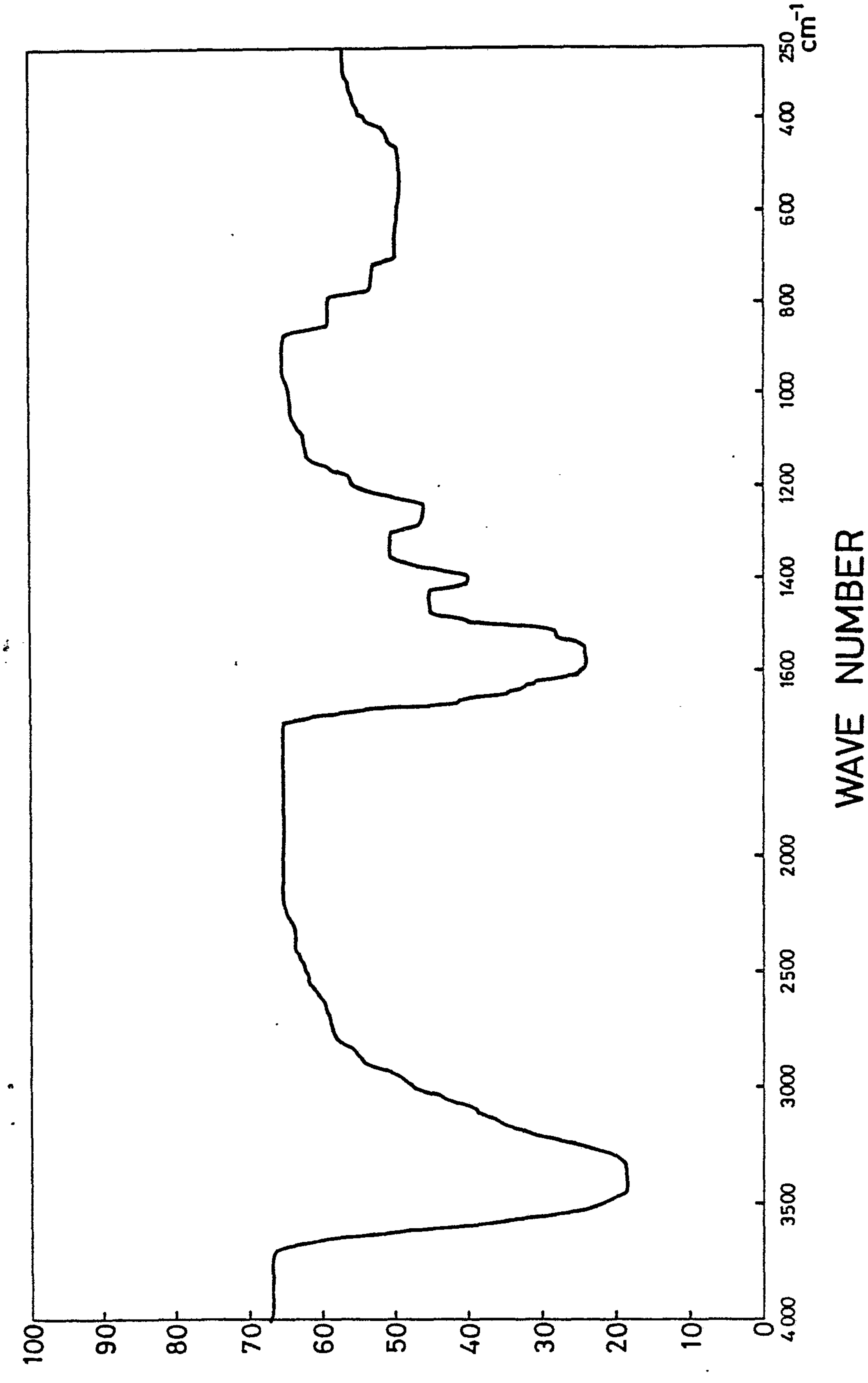

100
90
80
70
60
50
40
30
20
10
0
4000
3500
3000
2500
2000
1600
1400
1200
1000
800
600
400
250
cm⁻¹
WAVE NUMBER